# EUROPEAN PATENT APPLICATION

(11) **EP 3 255 149 A2**
(43) Date of publication of application: **13.12.2017**
(21) Application number: 17173216.7
(22) Date of filing: 02.05.2007
(51) Int. Cl.: C12N 15/63, C12N 15/74, C12N 15/81

(54) **MICROBIAL INTESTINAL DELIVERY OF OBESITY RELATED PEPTIDES**

(30) Priority: 02.05.2006 EP 06113388
(62) Divisional of application: 07728719.1
(71) Applicant: Intrexon Actobiotics NV, 9052 Zwijnaarde (BE)
(72) Inventor: Rottiers, Pieter, 9840 De Pinte (BE)
(74) Representative: Pohlman, Sandra M.

(57) **Abstract**

The present invention relates to the microbial delivery of obesity related peptides. More specifically, the invention relates to the use of genetically modified yeasts and/or lactic acid bacteria for the delivery of neuropeptides and/or peptide hormones that play a role in stimulation or inhibition of food intake and/or energy homeostasis.

## Description

The present invention relates to the microbial delivery of obesity related peptides. More specifically, the invention relates to the use of genetically modified yeasts and/or lactic acid bacteria for the delivery of neuropeptides and/or peptide hormones that play a role in stimulation or inhibition of food intake and/or energy homeostasis.

Eating disorders such as anorexia nervosa, bulimia nervosa and Binge eating disorder affect a significant part of the population. Obesity is one of the most common metabolic diseases and one of the greatest threats of public health, because of the numerous complications associated with it, such as diabetes, hypertension and cardiovascular diseases.

In view of the increase of overweight world-wide, the neuro-hormonal control of food intake and energy expenditure becomes an important medical issue. Recently, studies have been published on obesity related peptides, having either a stimulating, or inhibiting effect on food intake, and their possible use in body weight control (Broberger, 2005; Konturek *et al.,* 2005; Stanley *et al.,* 2005).

Obesity related peptides are normally active in the central nervous system. In a medical situation, the compounds are normally applied by parenteral injection. However, the effect of obesity related peptides is a temporary shift of the balance, and in most cases, at least a daily injection is needed for a stabilization of the disorder. This form of application is very inconvenient for the patient, and intensive research towards other forms of application, such as nasal, buccal, rectal or oral has been carried out. Especially the oral application is easier and better accepted by the patients. However, the main drawback of the oral application is that the obesity related peptide needs to pass to the gastro-intestinal tract, where they are normally inactivated by the high acidity of the stomach and digested by the proteolytic enzymes present in the gastro-intestinal tract. This makes that even proteins that are incapsulated for intestinal delivery are rather inefficient and need to be delivered in high doses. To overcome this problem, absorption enhances have been proposed. Such enhancers are, amongst others, described in WO 02/28436, WO 04/104018 and WO 05/112633 and have been described for oral delivery of obesity related peptides such as insulin, Glucagon like peptide-1 and Peptide YY.

Although delivery enhancers may be useful in certain cases, the combined microencapsulation, needed for the stabilization of the obesity related peptide, and use of an enhancer to increase the bioavailability of the obesity related peptide complicates the formulation of the medicament.

Intestinal microbial delivery is known to the person skilled in the art and has been described in several applications (amongst others WO 97/14806, WO 00/23471, WO 01/02570). However, till now, the successful applications have been limited to local delivery of peptides in a damaged gut. There are no indications in the art that microbial delivery can be used for systemic delivery of compounds that may need to pass the blood brain barrier, such as obesity related peptides, and no data are available on the uptake of peptides in the intact gut. Surprisingly we found that when using microbial delivery, by bacteria or yeast, contrary to classical microencapsulation for intestinal delivery, there is no need for the use of an enhancer or delivery aid. Even more surprisingly, the bioavailability of microbial delivered protein to the intact gut seems to be higher than that of directly delivered protein, such a protein delivered by intragastric injection.

A first aspect of the invention is the use of a genetically modified organism for the intestinal delivery of obesity related peptides.

In an embodiment, the genetically modified organism may be bacteria, preferably non-pathogenic and non-invasive bacteria, more preferably Gram-positive bacteria, and still more preferably lactic acid bacteria. In another embodiment, the genetically modified organism may be yeasts, preferably non-pathogenic and non-invasive yeasts. Hence, preferably, said genetically modified organism is selected from the group consisting of lactic acid bacteria and yeasts. Lactic acid bacteria comprise, but are not limited to *Lactobacillus* spp., *Carnobacterium* spp., *Lactococcus* spp., *Streptococcus* spp., *Pediococcus* spp., *Oenococcus* spp., *Enterococcus* spp. and *Leuconostoc* spp. Yeasts comprise, but are not limited to *Saccharomyces* spp., *Hansenula* spp., *Kluyveromyces* spp. *Schizzosaccharomyces* spp., *Zygosaccharomyces* spp., *Pichia* sp., *Monascus* spp., *Geotrichum* spp. and *Yarrowia* spp.

A preferred embodiment is the use of a genetically modified organism wherein the genetically modified organism is chosen from the group consisting of *Lactobacillus* spp., *Carnobacterium* spp., *Lactococcus* spp., *Streptococcus* spp., *Pediococcus* spp., *Oenococcus* spp., *Enterococcus* spp. and *Leuconostoc* spp; more preferably chosen from the group consisting of *Lactobacillus* spp and *Lactococcus* spp.; even more preferably chosen from the group consisting of *Lactococcus* spp.; and most preferably is *Lactococcus lactis.* Another preferred embodiment is the use of a genetically modified organism wherein the genetically modified organism is chosen from the group consisting of *Saccharomyces* spp., *Hansenula* spp., *Kluyveromyces* spp. *Schizzosaccharomyces* spp. *Zygosaccharomyces* spp., *Pichia* sp., *Monascus* spp., *Geotrichum* spp and *Yarrowia* spp.; more preferably chosen from the group consisting of *Saccharomyces* spp. and *Pichia* spp.; even more preferably chosen from the group consisting of *Saccharomyces* spp.; and most preferably is *Saccharomyces cerevisiae.*

Obesity related peptides are known to the person skilled in the art, and include, but are not limited to: 1) Agouti-related peptide, 2) Amylin, 3) Anorectin, 4) Bombesin, 5) Brain derived neural factor, 6) Calcitonin-gene related peptide, 7) Cholecystokinin, 8) Cocaine- and amphetamine-regulated transcript peptide, 9) Ciliary neurotrophic factor, 10) Corticotropin-releasing hormone, 11) Dynorphin, 12) β-endorphin, 13) Enterostatin, 14) Exendin, 15) Galanin, 16) Galanin like peptide, 17) Gastric inhibitory peptide, 18) Ghrelin, 19) Glucagon-like peptide-1, 20) Growth hormone releasing hormone, 21) Hypocretin/orexin, 22) Insulin, 23) Insulin like growth factor-I, 24) Insulin like growth factor-II, 25) Interleukin-1, 26) Peptide YY, 27) Leptin, 28) Melanin concentrating hormone, 29) Motilin, 30) Neuromedin B, 31) Neuromedin U, 32) Neuropeptide B, 33) Neuropeptide K, 34) Neuropeptide S, 35) Neuropeptide W, 36) Neuropeptide Y, 37) Neurotensin, 38) Oxytocin, 39) Prolactin releasing peptide, 40) Pro-opiomelanocortin and melanocortins derived thereof, 41) Somatostatin, 42) Thyrotropin-releasing hormone, 43) Urocortin, 44) VGF, 45) 26RFa, 46) Apolipoprotein A-IV, 47) Oxyntomodulin, 48) Pancreatic polypeptide, 49) Gastrin-releasing peptide, 50) Neuromedin, 51) Glucose-dependent insulinotrophic polypeptide, 52) Obestatin and 53) Growth hormone fragment (hGH₁₇₇₋₁₉₁).

Hence, in an embodiment of the use of a genetically modified organism for the intestinal delivery of obesity related peptides, the obesity related peptide is chosen from the group consisting of peptides listed under 1) to 53) above. In another embodiment, the obesity related peptide is chosen from the group consisting of peptides listed under 1) to 45) above.

In a further embodiment, the obesity related peptide is chosen from the group consisting of peptides listed under 1) to 21) and 23) to 53) above, or chosen from the group consisting of peptides listed under 1) to 21) and 23) to 45) above. In another embodiment, the obesity related peptide is chosen from the group consisting of peptides listed under 1) to 24) and 26) to 53) above, or chosen from the group consisting of peptides listed under 1) to 24) and 26) to 45) above. In a further embodiment, the obesity related peptide is chosen from the group consisting of peptides listed under 1) to 40) and 42) to 53) above, or chosen from the group consisting of peptides listed under 1) to 40) and 42) to 45) above. In a further embodiment, the obesity related peptide is chosen from the group consisting of peptides listed under 1) to 21), 23), 24), 26) to 40) and 42) to 53) above, or chosen from the group of peptides listed under 1) to 21), 23), 24), 26) to 40) and 42) to 45) above.

The above listed obesity related peptides and their physiological effects are generally known to a skilled person, who can choose a suitable peptide for delivery in a particular condition. By means of illustration, when the aim is to reduce food intake and/or decrease body weight, an obesity related peptide can be delivered that reduces appetite, reduces nutrient absorption and/or increases nutrient catabolism, etc.; when the aim is to enhance food intake and/or increase body weight, an obesity related peptide can be delivered that increases appetite, increases nutrient absorption and/or increases nutrient reserves, etc.

In an exemplary, non-limiting embodiment, when aiming at reducing food intake and/or body weight, the delivered obesity related peptide may be chosen from the group consisting of peptides listed under 2) to 10), 13),14) 16), 17), 19), 22) to 27), 29) to 33), 35), 37) to 43), 46), 47), 49) to 53) above; or, in another embodiment, may be chosen from the group consisting of peptides listed under 2) to 10), 13), 14), 16), 17), 19), 23), 24), 26), 27), 29) to 33), 35), 37) to 40), 42), 43), 46), 47), 49) to 53) above. It has been realised that obesity related peptides from these groups may display considerable anorexigenic effect when delivered by the genetically modified organisms of the invention. Preferably, said obesity related peptide is peptide YY (PYY) or exendin, even more preferably PYY or exendin-4.

In another exemplary, non-limiting embodiment, when aiming at increasing food intake and/or body weight, the delivered obesity related peptide may be chosen from the group consisting of peptides listed under 1), 11), 12), 15), 18) 20), 21), 28), 36), 44), 45) and 48) above. It has been realised that obesity related peptides from this groups may display considerable orexigenic effect when delivered by the genetically modified organisms of the invention.

As already noted, it is surprising that the above obesity related peptides can be efficiently delivered by genetically modified organisms. Indeed, most of these peptides are highly unstable in the intestine. Moreover, due to their peptidic nature, efficient entry of these peptides into the organism through intact intestine (which includes physical and biochemical barriers, such as, e.g., epithelial cell lining, the mucus layer and luminal and epithelial degradative enzymes, which protect the organism against the entry of, *interalia,* proteinaceous or peptidic toxins, pathogens or antigens) would be unexpected. Also, the successful demonstrations of microbial delivery has been so far primarily centred on local delivery of peptides in a damaged gut rather than a (sub)mucosal delivery through intact intestine.

Even more surprisingly, great majority of the delivered peptides are (neuro)peptides, that are normally synthesised and/or exert their effects in central or peripheral nervous system or in neuroendocrine tissues. It is entirely unexpected that such (neuro)peptides can still exert their physiological effects in the relevant tissues when delivered intestinally by the present genetically modified organisms. For example, preferred peptides from the above list which are normally synthesised and/or exert their effects in central or peripheral nervous system or in neuroendocrine tissues include peptides listed under 1) to 3), 5) to 12), 14) to 16), 18) to 21), 26) to 28), 30) to 40), 42) to 45), 52) and 53) above, albeit are not limited thereto.

In an embodiment, a genetically modified organism of the invention may deliver one obesity related peptide. It is however also contemplated that the genetically modified organism may deliver two or more, e.g., two, three, four or more, preferably two or three, more preferably two, different obesity related peptides as defined above. For example, in an embodiment, said organism may deliver two or more different orexigenic peptides as defined above. In another embodiment, the organism may deliver two or more different anorexigenic peptides as defined above.

It shall be appreciated that when two or more different obesity related peptides are delivered by the genetically modified organism, said peptides may achieve an additive or synergic physiological effect(s) in a subject, e.g., an additive or synergic decrease or increase in food intake and/or body weight. In a preferred embodiment, the genetically modified organism may deliver two or more obesity related peptides which achieve a synergic physiological effect in the subject. By means of a preferred, albeit non-limiting example, a genetically modified organism of the invention may deliver PYY and exendin, more preferably PYY and exendin-4, which together can act synergically, such that the achieved anorexigenic effect when delivered together is significantly greater than the sum of the observed individual effects when delivered alone.

The terms *peptide, protein* and *polypeptide* as used in this application are interchangeable. *Peptide* refers to a polymer of amino acids and does not refer to a specific length of the molecule. This term also includes post-translational modifications of the polypeptide, such as glycosylation, phosphorylation, amidation and acetylation.

In cases where the naturally occurring obesity related peptide is amidated, such as for glucagon like peptide-1, the peptide produced by the genetically modified organism is preferably not amidated.

In a further development of the invention, the genetically modified organism may deliver a binding molecule that binds to an obesity related peptide as defined herein. Advantageously, by binding to an endogenous obesity related peptide, a binding molecule may increase (agonist) or decrease (antagonist) the biological or physiological effects of said endogenous obesity related peptide in a subject. Accordingly, an aspect of the invention provides the use of a genetically modified organism for the intestinal delivery of a binding molecule capable of binding to an obesity related peptide as disclosed herein. In preferred embodiments, the biological or physiological effect of so bound obesity related peptide in a subject is increased or decreased.

In a yet another development of the invention, the genetically modified organism may deliver a binding molecule that binds to an endogenous receptor for an obesity related peptide as defined herein. Advantageously, by binding to said endogenous receptor, the binding molecule may increase (agonist) or decrease (antagonist) the biological activity of said receptor, and thereby mimic the presence or absence of the cognate obesity related peptide, respectively. Accordingly, an aspect of the invention provides the use of a genetically modified organism for the intestinal delivery of a binding molecule capable of binding to an endogenous receptor for an obesity related peptide as disclosed herein. In preferred embodiments, the biological activity of the so bound receptor in a subject is increased or decreased.

The term "molecule" in the expression "binding molecule" broadly refers to any chemical (e.g., inorganic or organic), biochemical or biological substance, molecule or macromolecule (e.g., biological macromolecule) or a combination or mixture thereof. Preferred binding molecules may include, without limitation, peptides, polypeptides or proteins, peptidomimetics, antibodies and fragments and derivatives thereof, aptamers, chemical substances, carbohydrates, polysaccharides, etc.

The term "binding" as used herein generally refers to a physical association, preferably herein a non-covalent physical association, between molecular entities, e.g., between a binding molecule and an obesity related peptide. In preferred embodiments, the binding molecule is capable of binding to the native conformation of the obesity related peptide or of the endogenous receptor for an obesity related peptide.

In a preferred embodiment, a binding molecule can bind to an obesity related peptide with high affinity. As used herein, binding can be considered "high affinity" when the affinity constant (K_{A}) of such binding is K_{A} > 1×10⁴ M⁻¹, preferably K_{A} ≥ 1×10⁵ M⁻¹, even more preferably K_{A} ≥ 1×10⁶ M⁻¹ such as, e.g., K_{A} ≥ 1×10⁷ M⁻¹, yet more preferably K_{A} ≥ 1×10⁸ M⁻¹, even more preferably K_{A} ≥ 1×10⁹ M⁻¹, e.g., K_{A} ≥ 1×10¹⁰ M⁻¹, and most preferably K_{A} ≥ 1×10¹¹ M⁻¹, e.g., K_{A} ≥ 1×10¹² M⁻¹, K_{A} ≥ 1×10¹³ M⁻¹, K_{A} ≥ 1×10¹⁴ M⁻¹, K_{A} ≥ 1×10¹⁵ M⁻¹ or even higher, wherein K_{A} = [binding partner 1_binding partner 2]/[binding partner 1][binding partner 2]. Determination of K_{A} can be carried out by methods known in the art, such as, e.g., using equilibrium dialysis and Scatchard plot analysis.

In a preferred embodiment, the binding of a binding molecule to an obesity related peptide, or to the endogenous receptor for an obesity related peptide is specific. The terms "specifically bind" and "specific binding" reflect a situation when a binding molecule binds to the respective obesity related peptide, or to the endogenous receptor for an obesity related peptide more readily than it would bind to a random, unrelated substance, such as another biological substance. For example, a binding molecule specifically binding to a given obesity related peptide ("Peptide 1"), or to an endogenous receptor for a given obesity related peptide ("Receptor 1") preferably displays little or no binding to other polypeptides, including other obesity related peptides or receptors therefor, under conditions where the binding molecule would bind with high affinity to said obesity related peptide ("Peptide 1"), or to said endogenous receptor for a given obesity related peptide ("Receptor 1"), respectively. Under little or no binding is meant K_{A} ≤ 1≤10⁴ M⁻¹, preferably K_{A} ≤ 1×10³ M⁻¹, more preferably K_{A} ≤ 1×10² M⁻¹, yet more preferably K_{A} ≤ 1×10¹ M⁻¹, e.g., K_{A} ≤ 1 M⁻¹, most preferably K_{A} << 1 M⁻¹, e.g., K_{A} ≤ 1×10⁻¹ M⁻¹, K_{A} ≤ 1×10⁻² M⁻¹, K_{A} ≤ 1×10⁻³ M⁻¹, K_{A} ≤ 1×10⁻⁴ M⁻¹, K_{A} ≤ 1×10⁻⁵ M⁻¹, K_{A} ≤ 1×10⁻⁶ M⁻¹, or smaller.

A binding molecule which can increase the biological or physiological effects of an obesity related peptide, or which can increase the biological activity of an endogenous receptor for an obesity related peptide is herein termed "activator" or "agonist". A binding molecule which can reduce, partially or completely, the biological or physiological effects of an obesity related peptide, or which can reduce, partially or completely, the biological activity of an endogenous receptor for an obesity related peptide is herein termed "inhibitor" or "antagonist". Typically, biological and physiological effects of an obesity related peptide as used herein denote to its orexigenic or anorexigenic effects in a subject. Biological activity of a receptor of for an obesity related peptide may denote, e.g., the effect of an activated receptor on downstream signalling pathways, e.g., activation or repression thereof, or on cell membrane potential, etc.

Any and all mechanisms by which the binding of a binding molecule to an obesity related peptide may enhance or decrease the physiological effects of said peptide in a subject are contemplated by the invention. By means of example and not limitation, such activators or inhibitors may, respectively, increase or decrease the stability of an obesity related peptide; reduce or promote degradation or turnover of an obesity related peptide; increase or reduce the interaction of an obesity related peptide with its cognate receptor, target cells or tissues; etc.

Any and all mechanisms by which the binding of a binding molecule to the endogenous receptor for an obesity related peptide may enhance or decrease the biological activity of said receptor in a subject are contemplated by the invention. Typically, the binding of agonists will activate the receptor, *i.e*., such agonists may mimic the binding of an obesity related peptide to its cognate receptor. Typically, antagonists may bind to a receptor in a non-productive way, *i.e*., such binding does not activate the receptor, and can preferably prevent binding of the respective obesity related peptide to its receptor and/or any concurrent activation of the receptor, e.g., in a competitive or non-competitive way.

As can be appreciated, delivery of agonists of orexigenic peptides (e.g., ghrelin, melanin, etc.) or their receptors and/or antagonists of anorexigenic peptides (e.g., GLP-1, PYY and oxyntomodulin) or their receptors will have a stimulating effect on food intake and/or body weight, while delivery of antagonists of orexigenic peptides or their receptors and/or agonists of anorexigenic peptides or their receptors will diminish food intake and/or body weight.

In a particularly preferred embodiment, a binding molecule as contemplated herein may be an antibody.

As used herein, the term "antibody" broadly refers to any immunologic binding agent, whether natural or partly or wholly engineered. The term specifically encompasses intact antibodies, including monovalent and/or mono-specific antibodies, and multivalent (e.g., 2-, 3- or more-valent) and/or multi-specific antibodies (e.g., bi- or more-specific antibodies) formed from at least two intact antibodies, and further includes antibody fragments insofar they exhibit the ability to specifically bind an antigen of interest (i.e., a given obesity related peptide or receptor therefor, or a given enzyme as disclosed herein), as well as multivalent and/or multi-specific composites of such antibody fragments. The term "antibody" further includes any polypeptide which is made to encompass at least one complementarity-determining region (CDR) capable of specifically binding to an epitope on an antigen of interest. In an embodiment, the antibody may be any of IgA, IgD, IgE, IgG or IgM immunoglobulin isotypic class or subclass thereof, and preferably IgG immunoglobulin class or subclass thereof.

In some instances, e.g., in certain immunoglobulin molecules derived from camelid species or engineered based on camelid immunoglobulins, a complete immunoglobulin molecule may consist of heavy chains only, with no light chains (see, e.g., Hamers-Casterman et al. 1993. Nature 363: 446-448; WO 94/04678). In these immunoglobulins the heavy chain variable region, referred to as VHH, forms the entire CDR. Such heavy-chain immunoglobulin molecules naturally devoid of light chains, and functional fragments and/or derivatives thereof comprising or consisting essentially of the VHH domain or a functional portion thereof, are also included by the term "antibody" as used herein, and constitute a preferred embodiment thereof. A method for producing bivalent or multivalent single domain antibodies, i.e. VHH polypeptide constructs, is disclosed in WO 96/34103.

Further preferred embodiments of antibodies include, without limitation, chimeric antibodies (see, e.g., US 4,816,567 and Morrison et al. 1984. PNAS 81: 6851-6855 for guidance), primatised antibodies and humanised antibodies (see, e.g., Jones et al. 1986. Nature 321: 522-525; Riechmann et al. 1988. Nature 332: 323-329; and Presta 1992. Curr Op Struct Biol 2: 593-596 for guidance).

In further embodiments, the invention may employ antibody fragments, which can display advantages, such as, e.g., smaller size, easier delivery, absence of effector domains, etc.

"Antibody fragments" comprise a portion of an intact antibody, comprising the antigen-binding or variable region thereof. Examples of antibody fragments include Fab, Fab', F(ab')2, Fv and scFv fragments; diabodies; triabodies; single-chain antibody molecules; and multivalent and/or multi-specific antibodies formed from antibody fragment(s). The above designations Fab, Fab', F(ab')2, Fv, scFv etc. are intended to have their art-established meaning.

By means of further explanation, papain digestion of antibodies produces two identical antigen-binding fragments, called "Fab" fragments, each with one antigen-binding site, and a residual "Fc" fragment. Pepsin treatment yields an F(ab')2 fragment that has two antigen-binding sites. A typical Fab fragment also contains the constant domain of the light chain and the first constant domain (CH1) of the heavy chain. Fab' fragments differ from Fab fragments by the addition of a few residues at the C-terminus of the heavy chain CH1 domain including one or more Cys residues from the antibody hinge region.

"Fv", which also constitute a preferred embodiment, is an antibody fragment which contains a complete antigen-recognition and antigen-binding site. This region consists essentially of a dimer of one heavy chain and one light chain variable domain in tight, non-covalent association. It is in this configuration that the three hypervariable regions of each variable domain interact to define an antigen-binding site on the surface of the VH-VL dimer. Collectively, the six hypervariable regions confer antigen-binding specificity to the antibody. However, even a single variable domain, VH or VL, i.e., half of an Fv comprising only three hypervariable regions specific for an antigen, has the ability to recognise and bind antigen, although at a lower affinity than the entire binding site ("single-domain antibodies").

"Single-chain Fv" or "scFv" antibody fragments, constituting a further preferred embodiment, comprise the VH and VL domains of antibody, wherein these domains are present in a single polypeptide chain. Preferably, the Fv polypeptide further comprises a polypeptide linker between the VH and VL domains which enables the scFv to form the desired structure for antigen binding (see, e.g., Plückthun in The Pharmacology of Monoclonal Antibodies, vol. 113, Rosenburg and Moore eds., Springer-Verlag, New York, pp. 269-315, 1994, for guidance).

Moreover, the term "Fv" also encompasses further functional (i.e., specifically antigen-binding) fragments thereof. Examples of such fragments include but are not limited to a "minibody" which comprises a fragment of the heavy chain only of the Fv, a "microbody" which comprises a small fractional unit of antibody heavy chain variable region (see PCT/IL99/00581), similar bodies having a fragment of the light chain, and similar bodies having a functional unit of a light chain variable region. It shall be appreciated that a fragment of an Fv molecule can be a substantially circular or looped polypeptide.

Bi-valent and/or bi-specific antibodies comprising scFv molecules, constituting a further preferred embodiment, can be constructed, for instance, by genetic coupling of both scFv molecules through a polypeptide linker (see, e.g., US 5,091,513 and US 5,637,481). When this linker contains a heterodimerising helix, a tetravalent bi-specific antibody is formed.

"Diabodies", which also represent a preferred embodiment, refer to small antibody fragments with two antigen-binding sites, which fragments comprise a variable heavy domain (VH) connected to a variable light domain (VL) in the same polypeptide chain (VH-VL). By using a linker that is too short to allow pairing between the two domains on the same chain, the domains are forced to pair with the complementary domains of another chain and create two antigen-binding sites. Bivalent diabodies show dramatically reduced dissociation rates (Koff) as compared to the parental scFv molecules. Diabodies are described more fully in, for example, EP 404,097, WO 93/11161, and Hollinger et al. 1993 (PNAS 90: 6444-6448). Production of bi- or more-specific diabodies is described, e.g., in WO 02/02781.

Shortening of the linker between VH and VL domains to < 1-2 Angstrom promotes formation of a trimeric molecule, i.e. a triabody, also constituting a preferred embodiment. The triabody structure may be used as a blueprint for the design and construction of trivalent and/or tri-specific antibody fragments (e.g. by linking the heavy and light chain V-domains of three different antibodies A , B and C to form two different chains VHA-VLB , VHB-VLC and VHC-VLA). Triabodies could bind three different or identical epitopes on the same molecule leading to very high apparent affinities.

A skilled person will understand that an antibody can include one or more amino acid deletions, additions and/or substitutions (e.g., conservative substitutions), insofar such alterations preserve its binding of the respective antigen. An antibody may also include one or more native or artificial modifications of its constituent amino acid residues (e.g., glycosylation, etc.).

In a further preferred embodiment, a binding molecule as contemplated herein may be a dominant negative variant of an obesity related peptide. "Dominant negative variants" as used herein mean mutations that produce a peptide or protein that adversely affects the function, and thereby the biological or physiological effect, of the corresponding normal, wild-type obesity related peptide.

In a further preferred embodiment, a binding molecule as contemplated herein may induce or suppress the active secretion of an endogenous obesity related peptides.

Binding molecules of the invention, such as, for instance, antibodies and dominant negative variants of obesity relating peptides, can easily be expressed in the micro-organisms of the invention with the benefit of significant reduced production costs and without limitations in production capacity. Exemplary but non-limiting methods for bacterial delivery of antibodies have been disclosed in, e.g., WO 2007/025977.

A genetically modified organism of the invention may deliver one binding molecule capable of specific binding to one obesity related peptide (mono-specific) or to two or more different obesity related peptides (bi- or more-specific), e.g., preferably to only one obesity related peptide. A genetically modified organism of the invention may deliver one binding molecule capable of specific binding to one endogenous receptor for an obesity related peptide (mono-specific) or to two or more different endogenous receptors for the same or different obesity related peptides (bi- or more-specific), e.g., preferably to only receptor.

Alternatively, a genetically modified organism of the invention may deliver two or more, e.g., two, three four or more, preferably two or three, more preferably two, different binding molecules, which may be capable of binding to the same obesity related peptide or receptor, or may bind to two or more different obesity related peptides or receptors.

In a yet further development of the invention, a genetically modified organism may deliver an inhibitor of an endogenous enzyme that catalyses breakdown of nutrients in the gastrointestinal (GI) tract of a subject. Accordingly, an aspect of the invention provides use of a genetically modified organism for intestinal delivery of an inhibitor of an enzyme that catalyses breakdown of nutrients in the GI tract of a subject.

As can be appreciated, delivery of inhibitors of GI enzymes responsible for the breakdown of nutrients can decrease the availability and uptake of nutrients in the gut of a subject and thereby decrease the overall caloric intake. This can advantageously reduce the overall body weight of the subject.

In a preferred embodiment, said enzyme catalyses the breakdown of nutrients chosen from the group consisting of: polysaccharides, oligosaccharides, disaccharides, proteins, polypeptides, peptides and lipids. In a further preferred embodiment, said enzyme catalyses the breakdown of lipids, even more preferably of triglycerides.

Hence, in a preferred embodiment, said enzyme may be chosen from the group consisting of: pancreatic protease, preferably trypsin and chymotrypsin, pancreatic lipase, and pancreatic amylase. In a further preferred embodiment, the enzyme is pancreatic lipase.

In exemplary embodiments, the inhibitor may be an antagonistic antibody specifically binding to said enzyme, e.g., to pancreatic lipase, or may be a dominant negative variant of such enzyme. In a further exemplary embodiment, an inhibitor of pancreatic lipase may be lipstatin (Weibel et al. 1987. J Antibiot (Tokyo) 40: 1081-5).

It shall be appreciated that in the present invention the genetically modified organism may deliver either a) an obesity related peptide, or b) a binding molecule capable of binding to an obesity related peptide, or c) a binding molecule capable of binding to an endogenous receptor for an obesity related peptide, or d) an inhibitor of an enzyme that catalyses breakdown of nutrients in the gastrointestinal (GI) tract. Alternatively, the genetically modified organism may deliver a combination of any two or all of a), b), c) and d) as above.

When delivered in combination, the delivered substances may produce an additive or synergic, preferably synergic. Accordingly, an aspect of the invention provides the use of a genetically modified organism for the intestinal delivery of: a) an obesity related peptide and/or b) a binding molecule capable of binding to an obesity related peptide and/or c) a binding molecule capable of binding to an endogenous receptor for an obesity related peptide and/or d) an inhibitor of an enzyme that catalyses breakdown of nutrients in the gastrointestinal (GI) tract.

Another aspect of the invention is the use of a genetically modified organism producing: a) an obesity related peptide and/or or b) a binding molecule capable of binding to an obesity related peptide and/or c) a binding molecule capable of binding to an endogenous receptor for an obesity related peptide and/or d) an inhibitor of an enzyme that catalyses the breakdown of nutrients in the GI tract, as discussed herein before, as a medicament.

As used here, a genetically modified organism producing a) and/or b) and/or c) and/or d) as set out in the previous paragraph means that a host organism is transformed with at least DNA encoding, a) and/or b) and/or c) and/or d) as above, respectively, which results in the genetically modified organism. Producing a) and/or b) and/or c) and/or d) as above means that the genetically modified organism is producing a) and/or b) and/or c) and/or d) as above, respectively, when used as a medicament, i.e. the genetically modified organism is at least producing said a) and/or b) and/or c) and/or d) as above once the genetically modified organism delivered in the intestine. Said production can be judged directly, by measuring the local intestinal product concentration e.g. in an animal model, or it can be measured indirectly, either by increase of the product in the blood or by measuring the effect of the delivered a), b), c) or d) as above on the body.

Still another aspect is the use of a genetically modified organism producing: a) an obesity related peptide, and/or b) a binding molecule capable of binding to an obesity related peptide, and/or c) a binding molecule capable of binding to an endogenous receptor for an obesity related peptide and/or c) an inhibitor of an enzyme that catalyses the breakdown of nutrients in the GI tract, as discussed herein before, according to the invention, for the preparation of a medicament to treat obesity and/or diabetes and/or eating disorders such as anorexia nervosa, bulimia nervosa, or Binge eating disorder.

A further aspect is a method of preventing or treating obesity and/or diabetes and/or eating disorders such as anorexia nervosa, bulimia nervosa, or Binge eating disorder in a subject in need of such treatment, comprising administering to said subject a therapeutically effective amount of a genetically modified organism producing: a) an obesity related peptide, and/or b) a binding molecule capable of binding to an obesity related peptide, and/or c) a binding molecule capable of binding to an endogenous receptor for an obesity related peptide, and/or d) an inhibitor of an enzyme that catalyses the breakdown of nutrients in the GI tract, as discussed herein before.

The term "subject" encompasses in particular humans and animals. The animal may preferably be a mammal, such as, e.g., domestic animals, farm animals, zoo animals, sport animals, pet and experimental animals such as dogs, cats, guinea pigs, rabbits, rats, mice, horses, cattle, cows; primates such as apes, monkeys, orang-utans, and chimpanzees; canids such as dogs and wolves; felids such as cats, lions, and tigers; equids such as horses, donkeys, and zebras; food animals such as cows, pigs, and sheep; ungulates such as deer and giraffes; rodents such as mice, rats, hamsters and guinea pigs; and so on.

The term "therapeutically effective amount" refers to an amount of a therapeutic substance or composition effective to treat a disease or disorder in a subject, e.g., human or animal, i.e., to obtain a desired local or systemic effect and performance. While precise dosages cannot be defined for each and every embodiment of the invention, they will be readily apparent to those skilled in the art once armed with the present invention. The dosage could be determined on a case by case way by measuring the serum level concentrations of the delivered protein after administration of predetermined numbers of cells, using well known methods, such as those known as ELISA or Biacore. The analysis of the kinetic profile and half life of the delivered recombinant protein provides sufficient information to allow the determination of an effective dosage range for the genetically modified organism.

The micro-organism producing the a) an obesity related peptide and/or or b) a binding molecule capable of binding to an obesity related peptide and/or c) a binding molecule capable of binding to an endogenous receptor for an obesity related peptide and/or d) an inhibitor of an enzyme that catalyses the breakdown of nutrients in the GI tract, as discussed herein before, may be delivered in effective amounts per unit dose of at least 10⁴ colony forming units (cfu) to 10¹² cfu per day, preferably between 10⁶ cfu to 10¹² cfu per day, most preferably between 10⁹ cfu and 10¹² cfu per day. In accordance with the method as described in Steidler *et al.* (2000) or through ELISA, the delivered molecule of e.g. of 10⁹ cfu is secreted to at least 1 ng to 1 µg; the skilled person in the art can calculate the range of binding molecule in relation to any other dose of cfu.

In preferred embodiments, where the microorganism is administered at the above cfu doses, the level of expression of the delivered molecule(s) by said microorganism may amount to ≥0.1% of the total cellular protein of said microorganism, e.g., ≥0.5%, more preferably ≥1%, e.g., ≥2%, ≥3% or ≥4%, even more preferably ≥5%, e.g., ≥6%, ≥7%, ≥8% or ≥9%, and still more preferably ≥10%, e.g., ≥15% or even at ≥20% of the total cellular protein of said microorganism, as measured by, e.g., SDS-PAGE and Coomassie or silver staining.

The a) an obesity related peptide and/or or b) a binding molecule capable of binding to an obesity related peptide and/or c) a binding molecule capable of binding to an endogenous receptor for an obesity related peptide and/or d) an inhibitor of an enzyme that catalyses the breakdown of nutrients in the GI tract may be delivered in a dose of at least 10 fg to 100 µg per day, preferably between 1pg and 100 µg per day, most preferably between 1 ng and 100 µg per day.

Unit doses may be administered from thrice or twice each day to once every two weeks until a therapeutic effect is observed. It will be recognized, however, that lower or higher dosages and other administration schedules may be employed.

In a further aspect, the invention thus also provides a pharmaceutical composition comprising the genetically modified organism producing: a) an obesity related peptide, and/or b) a binding molecule capable of specifically binding to an obesity related peptide, and/or c) a binding molecule capable of binding to an endogenous receptor for an obesity related peptide, and/or d) an inhibitor of an enzyme that catalyses the breakdown of nutrients in the GI tract, as discussed herein before.

Preferably, such formulations comprise a therapeutically effective amount of said genetically modified organism of the invention and a pharmaceutically acceptable carrier, i.e., one or more pharmaceutically acceptable carrier substances and/or additives, e.g., buffers, carriers, excipients, stabilisers, etc.

The term "pharmaceutically acceptable" as used herein is consistent with the art and means compatible with the other ingredients of a pharmaceutical composition and not deleterious to the recipient thereof.

The genetically modified organism of the invention can be suspended in a pharmaceutical formulation for administration to a human or animal having the disease to be treated. Such pharmaceutical formulations include but are not limited to live genetically modified organism of the invention and a medium suitable for administration. The genetically modified organism may be lyophilized in the presence of common excipients such as lactose, other sugars, alkaline and/or alkali earth stearate, carbonate and/or sulfate (for example, magnesium stearate, sodium carbonate and sodium sulfate), kaolin, silica, flavorants and aromas.

Cells so-lyophilized may be prepared in the form of capsules, tablets, granulates and powders, each of which may be administered by the oral route.

Alternatively, some genetically modified organisms may be prepared as aqueous suspensions in suitable media, or lyophilized genetically modified organisms may be suspended in a suitable medium just prior to use, such medium including the excipients referred to herein and other excipients such as glucose, glycine and sodium saccharinate.

For oral administration, gastroresistant oral dosage forms may be formulated, which dosage forms may also include compounds providing controlled release of the host cells and thereby provide controlled release of the desired protein encoded therein. For example, the oral dosage form (including tablets, pellets, granulates, powders) may be coated with a thin layer of excipient (usually polymers, cellulosic derivatives and/or lipophilic materials) that resists dissolution or disruption in the stomach, but not in the intestine, thereby allowing transit through the stomach in favour of disintegration, dissolution and absorption in the intestine.

The oral dosage form may be designed to allow slow release of the host cells and of the recombinant protein thereof, for instance as controlled release, sustained release, prolonged release, sustained action tablets or capsules. These dosage forms usually contain conventional and well known excipients, such as lipophilic, polymeric, cellulosic, insoluble, swellable excipients. Controlled release formulations may also be used for any other delivery sites including intestinal, colon, bioadhesion or sublingual delivery (i.e., dental mucosal delivery) and bronchial delivery. When the compositions of the invention are to be administered rectally or vaginally, pharmaceutical formulations may include suppositories and creams. In this instance, the host cells are suspended in a mixture of common excipients also including lipids. Each of the aforementioned formulations are well known in the art and are described, for example, in the following references: Hansel et al. (1990, Pharmaceutical dosage forms and drug delivery systems, 5th edition, William and Wilkins); Chien 1992, Novel drug delivery system, 2nd edition, M. Dekker); Prescott et al. (1989, Novel drug delivery, J.Wiley & Sons); Cazzaniga et al, (1994, Oral delayed release system for colonic specific delivery, Int. J. Pharm.i08:7').

The above aspects and embodiments are further supported by the following examples which are in no instance to be considered limiting.

### BRIEF DESCRIPTION OF THE FIGURES

**Figure 1****:** Plasmid map of the vector pYES2T-ppMF
**Figure 2****:** Plasmid map of pYES2T-hPYY
**Figure 3****:** Plasmid map of vector pT1NX
**Figure 4****:** Plasmid map of pT1dmpPYY(3-36)
**Figure 5****:** Plasmid map of pT1 Exendin-4
**Figure 6****:** Effects of *Saccharomyces cerevisiae* transformed with pYES2T-hPYY (indicated as SC-hPYY) treatment on 4 hour food intake. *S.cerevisiae* transformed with the empty vector (pYES2T, invitrogen) is indicated as SC-YES2T. Mice (n=12 SC-hPYY and n=11 SC-YES2T) were 10 weeks old and 6 week on High fat diet.
**Figure 7****:** Effects of *Lactococcus lactis* transformed with pT1Exendin-4 (indicated as LL-Ex4) and *Lactococcus lactis* transformed with pT1dmpPYY(3-36) (indicated as LL-PYY) treatment on 4-hour food intake. *L. lactis* transformed with the empty vector is indicated as LL-pTrex. Mice were 8 weeks old and 4 week on High fat diet. P-value compared with LL-pTREX (n=10)
**Figure 8****:** Effects of LL-Ex4 and LL-PYY treatment on 24-hour body weight gain. Mice were 8 weeks old and 4 week on High fat diet. Plasmids are indicated as in figure 7.

### EXAMPLES

### Materials and methods to the examples

### Animals

Female C57BI/6 mice (10-12 wks, 25-30g) were housed five per cage, where they were allowed to acclimatize, without handling, for a minimum of 1 wk. They were provided with High Fat Mice chow and tap water *ad libitum.* Before experimentation, animals were acclimated to their caging conditions for 1 wk (housed one per cage) and received two intragastric saline inoculations in order to minimize stress on the study days.

### Assembly of synthetic gene PYY (human)

A synthetic codon-optimized for *L. lactis* human PYY (3-36) (amino acid sequence: IKPEAPGEDASPEELNRYYASLRHYLNLVTRQRY) was assembled using the oligo's
PYY(3-36) 1S (ATCAAACCAGAAGCTCCAGG),
PYY (3-36) 2S (ATCAAACCAGAAGCTCCAGGTGAAGATGCTTCACCAGAAG),
PYY (3-36) 2AS (CTTCTGGTGAAGCATCTTCACCTGGAGCTTCTGGTTTGAT),
PYY (3-36) 3S (TGAAGATGCTTCACCAGAAGAACTTAACCGTTACTACGCT),
PYY (3-36) 4S (AACTTAACCGTTACTACGCTTCACTTCGTCACTACCTTAA),
PYY (3-36) 4AS (TTAAGGTAGTGACGAAGTGAAGCGTAGTAACGGTTAAGTT),
PYY (3-36) 5S (TCACTTCGTCACTACCTTAACCTTGTTACACGTCAACGTT),
PYY (3-36) 6S (CCTTGTTACACGTCAACGTTACTAACTAGTAGATCC),
PYY (3-36) 6AS (GGATCTACTAGTTAGTAACGTTGACGTGTAACAAGG),
PYY (3-36) 7S (ACTAACTAGTAGATC),
PYY (3-36)-Spe-S (GTCAACGTTACTAACTAGTAGATCC), and
PYY (3-36)-Spe-AS (GGATCTACTAGTTAGTAACGTTGAC).

The assembled hPYY-Spel PCR fragment had a length of 114 bp, was purified on agarose and digested by the restriction enzymes *Spe*I. This PCR fragment was used for the Lactococcus constructs as well as for the yeast constructs.

### Construction of pYEST2-hPYY(3-36)

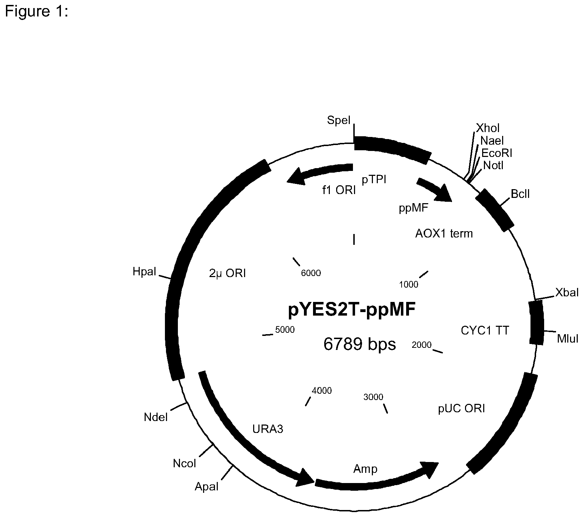

In pYES2, the GAL1 promoter has been replaced by the TPI promoter plus secretion signal ppMF, Nael and AOXI terminator as a stuffer, resulting in pYES2T-ppMF (Figure1)

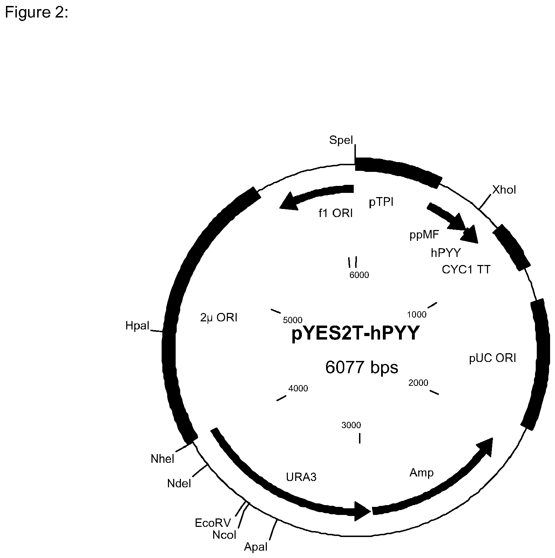

The pYEST2-ppMFwas digested by *Nae*I and *Xba*I. The DNA fragment was isolated and ligated with the hPYY-Spel PCR fragment. This resulted in a plasmid that was designated pYES2T-hPYY and contained the gene encoding human PYY(3-36) (figure 2). Heat competent MC1061 *E. coli* cells were transformed with the pYES2T-hPYY ligation mixture.

### Construction of hPYY secreting Saccharomyces cerevisiae

1 µg of the plasmid pYES2T-hPYY (prepared by Qiagen midi plasmid kit, Hilden, Germany; out of the *E. coli* strain MC1061[pYES2T-hPYY]) was electroporated into electrocompetent *Saccharomyces cerevisiae* INV Sc1 cells (Invitrogen™).

Saccharomyces cerevisiae INV Sc1 cells (Invitrogen™) is a strain that has a mating-α, his3Δ1, leu2-3,-112 trp1-289 and ura3-52 genotype. The transformed yeast cells were plated out on uracil deficient (selection) minimal medium (SD+CSM-U; 0,67% Yeast Nitrogen Base w/o Amino Acids (Difco, Detroit, MI) 2 % dextrose (Merck, Darmstadt, Germany) and 0.077% CSM-URA (Bio101 Systems, Morgan Irvine, CA)). One colony of the Saccharomyces strains *Saccharomyces cerevisisae* INV Sc1[pYES2T-hPYY] and the vector control Saccharomyces *cerevisisae* INV Sc1[pYES2] were respectively inoculated in 10 ml minimal uracil deficient medium (SD+CSM-U) and grown at 30°C under aerobic conditions. After 16 hours 10 ml fresh minimal uracil deficient medium was added and after an 32 hours the cells were pelleted by centrifugation (5 minutes@2500 tmp) and resuspended in YPD medium (YPD medium: 1% yeast extract, Difco; 2% dextrose, Merck; 2% peptone, Difco). After 16 hours the cells were pelleted by centrifugation and resuspended in 2ml YP (YPD without dextrose). For treatment, each mouse received 100 µL of this suspension by intragastric catheter.

### Assembly of synthetic gene Exendin-4

A synthetic codon-optimized for *L. lactis* Exendin-4 (amino acid sequence: HGEGTFTSDLSKQMEEEAVRLFIEWLKNGGPSSGAPPPS) was assembled using the oligo's Exendin4-1S (CACGGTGAAGGTACATTCAC),
Exendin4-2S (CACGGTGAAGGTACATTCACATCAGATCTTTCAAAACAAA),
Exendin4-2AS (TTTGTTTTGAAAGATCTGATGTGAATGTACCTTCACCGTG),
Exendin4-3S (ATCAGATCTTTCAAAACAAATGGAAGAAGAAGCTGTTCGT),
Exendin4-4S (TGGAAGAAGAAGCTGTTCGTCTTTTCATCGAATGGCTTAA),
Exendin4-4AS (TTAAGCCATTCGATGAAAAGACGAACAGCTTCTTCTTCCA),
Exendin4-5S (CTTTTCATCGAATGGCTTAAAAACGGTGGTCCATCATCAG),
Exendin4-6S (AAACGGTGGTCCATCATCAGGTGCTCCACCACCATCATAA),
Exendin4-6AS (TTATGATGGTGGTGGAGCACCTGATGATGGACCACCGTTT),
Exendin4-7S (GTGCTCCACCACCATCATAA),
Exendin4-Spe1 S (GGTGCTCCACCACCATCATAACTAGTGC),
Exendin4-Spe1-AS (GCACTAGTTATGATGGTGGTGGAGCACC).

The assembled Ex4-Spel PCR fragment had a length of 114 bp, was purified on agarose and digested by the restriction enzymes *Spe*I*.*

### Construction of pT1 Exendin-4 and pT1dmpPYY(3-36)

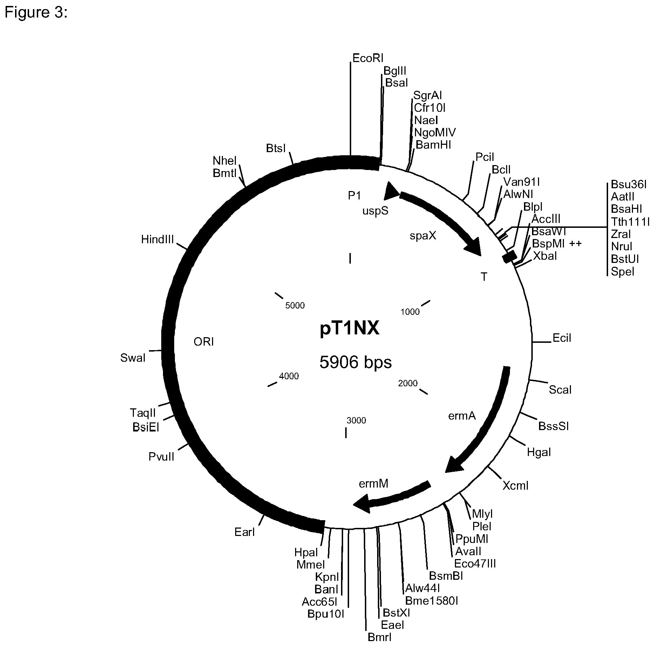

In pTREX, USP and spaX are inserted, resulting in pT1 NX (Figure 3).

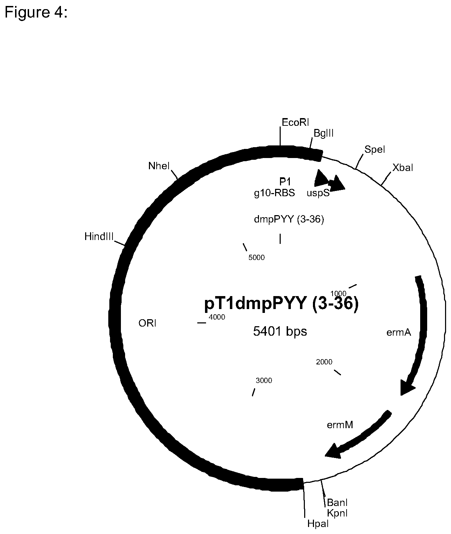

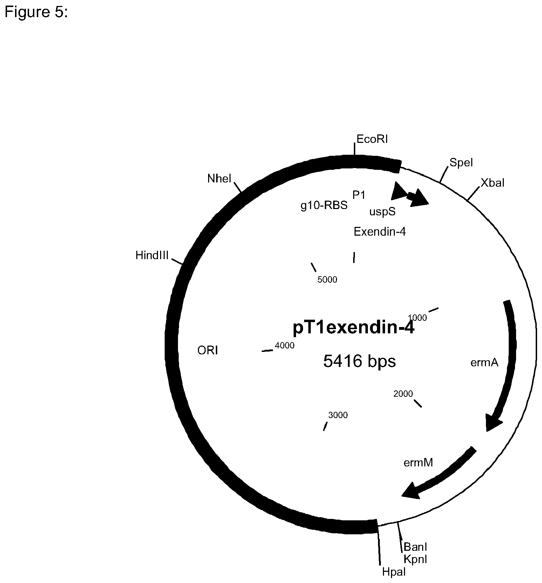

The pT1 NX was digested by *Nae*I and *Spe*I. The DNA fragment was isolated and ligated with the Ex4-Spel, respectively the Pyy-Spei PCR fragment. This resulted in a plasmid that was designated respectively pT1dmpPYY (figure 4) and pT1Exendin-4 (figure 5). Competent MG1363 *L.lactis* cells were transformed with the pT1Exendin-4 ligation mixture.

For intragastric inoculations, stock suspensions were diluted 1000-fold in fresh GM17 i.e. M17 (Difco Laboratories, Detroit, MI) supplemented with 0.5% glucose, and incubated at 30°C. After 16 hours when the cells reached a saturation density of 2 x 10⁹ colony-forming units (CFU) per mL, an equivalent volume of fresh GM17 was added and incubated for an additional 1 hour at 30°C.. Bacteria were harvested by centrifugation and concentrated 10-fold in BM9 medium without glucose. For treatment, each mouse received 100 µL of this suspension by intragastric catheter.

### Acute feeding studies

C57BI/6 mice were fasted in separate cages for 16-18 h with free access to water. On the experimental day, animals were intragastric inoculated with at t = -2 h and t = 0 h with 100 µl of either *Saccharomyces cerevisae* YES2T (SC-YES2T, empty vector control) or SC-hPYY for the *Saccharomyces* PYY test; *Lactococcus lactis* pTREX (empty vector) or L. lactis pT1dmpPYY for the *Lactococcus* PYY tests or *Lactococcus lactis* pTREX (empty vector) or L. lactis pT1exendin-4 for the exendin tests. Immediately after t = 0 h, chow was placed within the cage of preweighed mice, and food intake (FI) was determined at 1, 2, 3 and 4 h by measuring the difference between preweighed chow and the weight of chow remaining at the end of each time interval.

### Example 1: effect of intestinal yeast delivery of human PYY (3-36) on food intake by mice

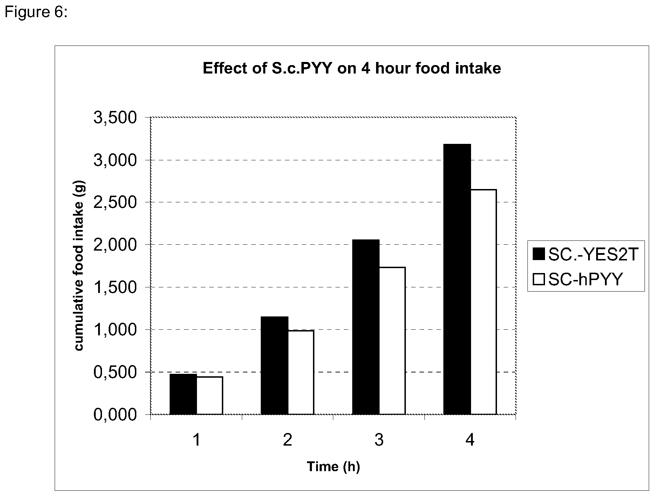

The bio-efficacy of S.c [pYES2T-hPYY] was evaluated by measuring post-dose food intake in mice. During the first four hours post-dose, food intake in the animals treated with S.c [pYES2T-hPYY] was 16% lower than in the animals dosed with placebo S.c [pYES2T] (figure 6). The results from this study demonstrate the feasibility of intestinal delivery of PYY (3-36) by S.c and present an opportunity to develop an oral dosage form of PYY (3-36) for the treatment of eating disorders and/or obesity.

### Example 2: effect on the food intake by mice of intestinal delivery of human PYY (3-36) and exendin-4 by lactic acid bacteria

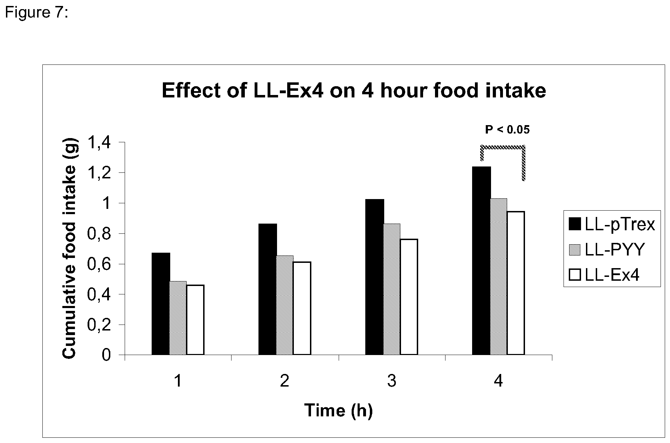

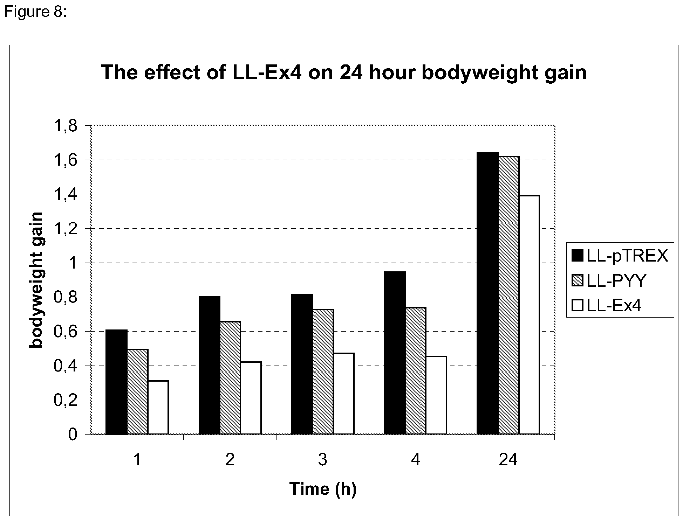

The bio-efficacy of LL-pT1-Exendin-4 and LL-pT1dmpPYY(3-36)was evaluated by measuring post-dose food intake in mice. During the first four hours post-dose, food intake in the animals treated with LL-pT1-Exendin-4 was 31% lower than in the animals dosed with placebo LL-pTREX (figure 7). The effect of intragastric inoculated LL-pT1-Exendin-4 on weight gain was evaluated in a 24-hour study. During the first 4 hours of the study, the weight gain in the group receiving Ex4 was almost 50 % lower than in the placebo group and 18% at the end of the study (figure 8). The results from these studies demonstrate the feasibility of intestinal delivery of Ex4 and present an opportunity to develop an oral dosage form of Ex4 for the treatment of eating disorders and/or obesity.

### REFERENCES

- Broberger, C. (2005). Brain regulation of food intake and appetite: molecules and networks. J. Int. Med. 258, 301-327.
- Konturek, P.C., Konturek, J.W., Czesnikiewicz-Guzik, M., Brzozowski, T., Sito, E. and Konturek, S.J. (2005). Neuro-hormonal control of food intake: basic mechanisms and clinical implications. J. Physiology Pharmacology 56, Supp 6, 5-25.
- Stanley, S., Wynne, K., McGowan, B. and Bloom, S. (2005). Hormonal regulation of food intake. Physiol. Rev. 85, 1131-1158.
- Steidler L, Hans W, Schotte L, Neirynck S, Obermeier F, Falk W, Fiers W, Remaut E (2000). Treatment of murine colitis by Lactococcus lactis secreting interleukin-10. Science 289(5483):1352-5.

In view of the foregoing, it will be appreciated that the invention described herein inter alia relates to the following items:
1. The use of a genetically modified organism for the intestinal delivery of: a) an obesity related peptide and/or b) a binding molecule capable of binding to an obesity related peptide and/or c) a binding molecule capable of binding to an endogenous receptor for an obesity related peptide and/or d) an inhibitor of an enzyme that catalyses breakdown of nutrients in the gastrointestinal (GI) tract.
2. The use of a genetically modified organism producing: a) an obesity related peptide and/or b) a binding molecule capable of binding to an obesity related peptide and/or c) a binding molecule capable of binding to an endogenous receptor for an obesity related peptide and/or d) an inhibitor of an enzyme that catalyses breakdown of nutrients in the GI tract, as a medicament.
3. The use of a genetically modified organism producing: a) an obesity related peptide and/or b) a binding molecule capable of binding to an obesity related peptide and/or c) a binding molecule capable of binding to an endogenous receptor for an obesity related peptide and/or d) an inhibitor of an enzyme that catalyses breakdown of nutrients in the GI tract, for the production of a medicament to treat eating disorders, or obesity.
4. The use according to any of the previous items, wherein said genetically modified organism is a yeast or a lactic acid bacterium.
5. The use according to item 4, wherein said genetically modified organism is *Saccharomyces cerevisiae.*
6. The use according to item 4, wherein said genetically modified organism is *Lactococcus lactis.*
7. The use according to any of the preceding claims wherein said obesity related peptide is selected from the group consisting of Agouti-related peptide, Amylin, Anorectin, Bombesin, Brain derived neural factor, Calcitonin-gene related peptide, Cholecystokinin, Cocaine- and amphetamine-regulated transcript peptide, Ciliary neurotrophic factor, Corticotropin-releasing hormone, Dynorphin, β-endorphin, Enterostatin, Exendin, Galanin, Galanin like peptide, Gastric inhibitory peptide, Ghrelin, Glucagon-like peptide-1, Growth hormone releasing hormone, Hypocretin/orexin, Insulin, Insulin like growth factor-I, Insulin like growth factor-II, Interleukin-1, Peptide YY (PYY), Leptin, Melanin concentrating hormone, Motilin, Neuromedin B, Neuromedin U, Neuropeptide B, Neuropeptide K, Neuropeptide S, Neuropeptide W, Neuropeptide Y, Neurotensin, Oxytocin, Prolactin releasing peptide, Pro-opiomelanocortin and melanocortins derived thereof, Somatostatin, Thyrotropin-releasing hormone, Urocortin, VGF, 26RFa, Apolipoprotein A-IV, Oxyntomodulin, Pancreatic polypeptide, Gastrin-releasing peptide, Neuromedin, Glucose-dependent insulinotrophic polypeptide, Obestatin and Growth hormone fragment (hGH₁₇₇₋₁₉₁).
8. The use according to item 7, wherein said genetically modified organism is a PYY producing S. *cerevisiae* strain.
9. The use according to item 7, wherein said genetically modified organism is an exendin-4 producing *L. lactis* strain.
10. The use according to any of items 1 to 6, wherein binding of the binding molecule to an obesity related peptide enhances the biological or physiological effect of said obesity related peptide in a subject.
11. The use according to any of items 1 to 6, wherein binding of the binding molecule to an obesity related peptide reduces the biological or physiological effect of said obesity related peptide in a subject.
12. The use according to any of items 1 to 6, 10 and 11, wherein the binding molecule capable of binding to an obesity related peptide is an antibody.
13. The use according to any of items 1 to 6 and 11, wherein the binding molecule capable of binding to an obesity related peptide is a dominant negative variant of said obesity related peptide.
14. The use according to any of items 1 to 6, wherein binding of the binding molecule to an endogenous receptor for an obesity related peptide enhances the biological activity of said receptor in a subject.
15. The use according to any of items 1 to 6, wherein binding of the binding molecule to an endogenous receptor for an obesity related peptide reduces the biological activity of said receptor in a subject.
16. The use according to any of items 14 or 15, wherein the binding molecule capable of binding to the endogenous receptor for an obesity related peptide is an antibody.
17. The use according to any of items 1 to 6, wherein the enzyme catalyses breakdown of nutrients chosen from the group consisting of: polysaccharides, oligosaccharides, disaccharides, proteins, polypeptides, peptides and lipids, preferably lipids, more preferably triglycerides.
18. The use according to any of items 1 to 6 or 17, wherein the enzyme is chosen from the group consisting of pancreatic protease, preferably trypsin or chymotrypsin, pancreatic lipase, and pancreatic amylase, more preferably the enzyme is pancreatic lipase.

## Claims

1. A genetically modified organism producing an obesity-related peptide for use in treating diabetes.

2. The genetically modified organism for the use according to claim 1, wherein said obesity-related peptide is selected from the group consisting of Agouti-related peptide, Amylin, Anorectin, Bombesin, Brain derived neural factor, Calcitonin-gene related peptide, Cholecystokinin, Cocaine- and amphetamine-regulated transcript peptide, Ciliary neurotrophic factor, Corticotropin-releasing hormone, Dynorphin, β-endorphin, Enterostatin, Exendin, Galanin, Galanin like peptide, Gastric inhibitory peptide, Ghrelin, Glucagon-like peptide-1, Growth hormone releasing hormone, Hypocretin/orexin, Insulin, Insulin like growth factor-I , Insulin like growth factor-II, Interleukin-1 , Peptide YY (PYY), Leptin, Melanin concentrating hormone, Motilin, Neuromedin B, Neuromedin U, Neuropeptide B, Neuropeptide K, Neuropeptide S, Neuropeptide W, Neuropeptide Y, Neurotensin, Oxytocin, Prolactin releasing peptide, Pro-opiomelanocortin and melanocortins derived thereof, Somatostatin, Thyrotropin- releasing hormone, Urocortin, VGF, 26RFa, Apolipoprotein A-IV, Oxyntomodulin, Pancreatic polypeptide, Gastrin-releasing peptide, Neuromedin, Glucose-dependent insulinotrophic polypeptide, Obestatin and Growth hormone fragment (hGH₁₇₇₋₁₉₁).

3. The genetically modified organism for the use according to claim 1 or claim 2, wherein said obesity peptide is a neuropeptide selected from the group consisting of: Agouti-related peptide, Amylin, Anorectin, Brain derived neural factor, Calcitonin-gene related peptide, Cholecystokinin, Cocaine- and amphetamine-regulated transcript peptide, Ciliary neurotrophic factor, Corticotropin- releasing hormone, Dynorphin, β-endorphin, Exendin, Galanin, Galanin like peptide, Ghrelin, Glucagon-like peptide-1 , Growth hormone releasing hormone, Hypocretin/orexin, Peptide YY (PYY), Leptin, Melanin concentrating hormone, Neuromedin B, Neuromedin U, Neuropeptide B, Neuropeptide K, Neuropeptide S, Neuropeptide W, Neuropeptide Y, Neurotensin, Oxytocin, Prolactin releasing peptide, Proopiomelanocortin and melanocortins derived thereof, Thyrotropin-releasing hormone, Urocortin, VGF, 26RFa, Obestatin and Growth hormone fragment (hGH₁₇₇₋₁₉₁).

4. The genetically modified organism for the use according to any one of claims 1 to 3, wherein the genetically modified organism produces glucagon-like peptide-1 or exendin-4.

5. The genetically modified organism for the use according to any one of claims 1 to 3, wherein the genetically modified organism produces peptide YY (PYY).

6. The genetically modified organism for the use according to any one of claims 1 to 5, wherein the genetically modified organism is a yeast or a lactic acid bacterium.

7. The genetically modified organism for the use according to claim 6, wherein the genetically modified organism is *Saccharomyces cerevisiae.*

8. The genetically modified organism for the use according to claim 6, wherein the genetically modified organism is *Lactococcus lactis.*

9. The genetically modified organism for the use according to claim 7, wherein the genetically modified organism is a PYY producing *Saccharomyces cerevisiae* strain.

10. The genetically modified organism for the use according to claim 8, wherein the genetically modified organism is an exendin-4 producing *L. lactis* strain.
